Europäisches Patentamt

European Patent Office

Office européen des brevets

(19)

(11) **EP 0 852 942 A1**

(12) **EUROPÄISCHE PATENTANMELDUNG**

(43) Veröffentlichungstag:
**15.07.1998  Patentblatt 1998/29**

(51) Int. Cl.$^6$: **A61K 7/13**

(21) Anmeldenummer: **97117920.5**

(22) Anmeldetag: **16.10.1997**

(84) Benannte Vertragsstaaten:
**AT BE CH DE DK ES FI FR GB GR IE IT LI LU MC NL PT SE**

(30) Priorität: **11.12.1996 DE 19651482**

(71) Anmelder:
**Wella Aktiengesellschaft**
**64274 Darmstadt (DE)**

(72) Erfinder:
• **Lenz, Uwe, Dr.**
  **64673 Zwingenberg (DE)**
• **Nowald, Ellen**
  **64367 Mühltal-Traisa (DE)**

(54) **Mittel zum Färben und Tönen von Haaren**

(57)    Gegenstand der Erfindung ist ein Mittel zum Färben und Tönen von Haaren, insbesondere menschlichen Haaren, auf der Basis von einem oder mehreren direktziehenden Farbstoffen, welches dadurch gekennzeichnet ist, daß es a) mindestens ein nichtionisches und/oder kationisches Polymer und b) mindestens ein anionisches Tensid oder eine Mischung aus mindestens einem anionischen und mindestens einem nichtionischen Tensid enthält.

EP 0 852 942 A1

Printed by Xerox (UK) Business Services
2.16.3/3.4

## Beschreibung

Die vorliegende Erfindung betrifft ein festigendes Mittel zum Färben und Tönen von menschlichen Haaren mit verbesserter Färbewirkung und gleichmäßigerem Auswaschverhalten.

Es ist allgemein bekannt, daß Haarfärbemittel auf der Basis von direktziehenden Farbstoffen ein sehr unterschiedliches Aufziehvermögen auf das menschliche Haar haben. So ist die Anlagerung der Farbstoffe in den porösen Haarstrukturen in der Regel wesentlich stärker als auf glatten Haaroberflächen.

Weiterhin benötigt man zum Erreichen des gewünschten Farbergebnisses in der Regel mehrere Farbstoffe, die in unterschiedlichen Mengenverhältnissen in die Färbemittel eingearbeitet werden. Durch Waschen der Haare werden die dort deponierten Farbstoffe teilweise wieder ausgewaschen. Bedingt durch das vorliegende Farbstoffgemisch werden einige Farbstoffe schneller ausgewaschen als andere. Dies bedeutet, daß sich der Farbcharakter des Haares in ungewünschter und nicht vorhersehbarer Weise verändert.

Es bestand daher ein Bedürfnis nach Haarfärbeprodukten auf der Basis von direktziehenden Farbstoffen, die die vorgenannten Nachteile nicht aufweisen.

Überraschenderweise wurde nunmehr gefunden, daß ein Haarfärbemittel auf der Basis von bestimmten direktziehenden Farbstoffen, welches eine Kombination aus nichtionischen und/oder kationischen Polymeren und nichtionischen und/oder anionischen Tensiden enthält, diese Aufgabe in hervorragender Weise löst.

Gegenstand der vorliegenden Anmeldung ist daher ein Mittel zum Färben und Tönen von Haaren, insbesondere menschlichen Haaren, auf der Basis von einem oder mehreren direktziehenden Farbstoffen, welches dadurch gekennzeichnet ist, daß es (a) mindestens ein nichtionisches und/oder kationisches Polymer und (b) mindestens ein anionisches Tensid oder eine Mischung aus mindestens einem anionischen und mindestens einem nichtionischen Tensid enthält.

Das erfindungsgemäße Mittel enthält mindestens einen direktziehenden Farbstoff, vorzugsweise jedoch mehrere direktziehende Farbstoffe, wobei die Gesamtmenge der direktziehenden Farbstoffe in der Regel 0,001 bis 5 Gewichtsprozent, insbesondere 0,05 bis 3 Gewichtsprozent, beträgt.

Als direktziehende Farbstoffe können übliche, physiologisch unbedenkliche, direktziehende Farbstoffe aus der Gruppe der Nitrofarbstoffe, Azofarbstoffe, Chinonfarbstoffe und Triphenylmethanfarbstoffe, wie zum Beispiel

4,N-Ethyl,N-(2'-hydroxyethyl)amino-1-(2''-hydroxyethyl)amino-2-nitro-benzol, 1-Amino-3-methyl-4-(2'-hydroxyethyl)amino-6-nitro-benzol, 2,2'-[(Amino-3-nitrophenyl)imino]-bisethanol Hydrochlorid (HC Red 13), 1-(2'-Hydroxyethyl)amino-2-nitro-4-bis-(2''-hydroxyethyl)amino-benzol, 4-Bis-(2'-Hydroxyethyl)amino-1-(2''-methoxyethyl)amino-nitrobenzol, 1-(2',3'-Dihydroxypropyl)amino-2-nitro-4-[methyl-(2''-hydroxyethyl)amino]-benzol, 1-[(2',3'-Dihydroxypropyl)amino]-2-nitro-4-[ethyl-2''-(hydroxyethyl)amino]-benzol, 1-(3'-Hydroxypropylamino)-2-nitro-4-bis-(2''-hydroxyethylamino)-benzol, 1-Amino-4-(2'-hydroxyethyl)-amino-nitrobenzol, 1-Hydroxy-2-amino-4,6-dinitro-benzol, 1-Amino-2-nitro-4-bis-(2'-hydroxyethyl)amino-benzol, 1-Amino-2-nitro-4-(2'-hydroxyethyl)amino-5-chlorbenzol, 1-(2'-Hydroxyethyl)amino-2-nitro-4-amino-benzol, 1-Hydroxy-3-nitro-4-amino-benzol, 1-(2'-Aminoethyl)amino-2-nitro-4-(2''-hydroxyethyl)-oxybenzol, 3-Nitro-4-(2'-hydroxyethyl)amino-phenylglycerinether, 1-Amino-5-chlor-4-(2',3'-dihydroxypropyl)amino-2-nitro-benzol, 1,4-Bis-[(2',3'-dihydroxypropyl)amino]-5-chlor-2-nitro-benzol, 1-Hydroxy-2-(2'hydroxyethyl)amino-4,6-dinitro-benzol, 2-Amino-6-chlor-4-nitrophenol, 1-Hydroxy-3-nitro-4-(3'-hydroxypropylamino)-benzol, 3-Nitro-4-ethylamino-benzoesäure, 4-Amino-2-nitro-diphenylamino-2-carbonsäure, 2-Chlor-6-ethylamino-4-nitrophenol, 2,5-Diamino-6-nitropyridin, 1,2,3,4-Tetrahydro-6-nitrochinoxalin, 4-(2'-Hydroxyethyl)amino-3-nitro-benzonitril, 4-(2'-Hydroxyethyl)amino-3-nitro-benzamid, 1-Amino-2-(2'-hydroxyethyl)amino-5-nitro-benzol, 1-Methoxy-2-(2'-hydroxyethyl)amino-5-nitro-benzol, 1-Hydroxy-3-nitro-4-(2'-hydroxyethyl)amino-benzol, 1-Hydroxy-2-amino-3-nitro-benzol, 1-Amino-2-methyl-6-nitro-benzol, 1-(2'Hydroxyethyl)-oxy-3-methylamino-4-nitro-benzol, 1-Methylamino-2-nitro-5-(2',3'-dihydroxypropyl)-oxybenzol, 1-(2'-Hydroxyethyl)amino-2-hydroxy-4-nitro-benzol, 1-Methoxy-3-(2'-aminoethyl)-amino-4-nitro-benzol, 1-(2'-Ureidoethyl)amino-4-nitro-benzol, 1-(2'-Hydroxyethyl)amino-2-nitro-benzol, 4-(2'-Hydroxyethyl)amino-3-nitro-trifluormethyl-benzol, 2,4-Bis-[N-(2'-hydroxyethyl)amino]-5-chlor-nitrobenzol, 4-(2',3'-Dihydroxypropyl)amino-3-nitro-trifluormethyl-benzol, 4-(2'-Hydroxymethyl)amino-3-nitro-methylbenzol, 4-(2'-Hydroxyethyl)amino-3-nitro-chlorbenzol, 1-(4'-Nitrophenylazo)-2-methyl-4-bis-(2''-hydroxyethyl)amino-benzol, 1-(3'-Nitro-4-amino)-phenylazo-2-hydroxy-7-trimethyl-ammoniumchlorid-naphthalin, 1-(2'Hydroxy-4'sulfo-6'nitro)-naphthylazo-2-hydroxynaphthalin, 1-(4'-Aminophenylazo)-2-methyl-4-bis-[(2'-hydroxyethyl)-amino]-benzol, 5-(4'-Dimethylaminophenylazo)-1,4-dimethyl-triazoniumchlorid, 1-(2'-Methoxyphenylazo)-2-hydroxy-7-trimethylammonium-naphthalinchorid, 1-(4'-Aminophenylazo)-2-hydroxy-7-trimethylammonium-naphthalin, 4-Hydroxy-3-[(4'-sulfo-1'-naphthyl)azo]-1-naphthalinsulfonsäure, 1-(4'-Sulfophenylazo)-2-hydroxynaphthalin, 1-(4'-Sulfonphenylazo)-2-hydroxy-6-sulfonaphthalin, 4-Amino-[4'-bis-(2''-hydroxyethyl)amino]azobenzol, 4-Amino-[4'-bis-(2''-hydroxyethyl)amino]-2'-methyl-azobenzol, 3-(2',6'-Diaminopyridyl-3'-azo)-pyridin, 7-Phenylazo-1-amino-3,6-disulfo-8-hydroxy-naphthalin, 5-Acetylamino-4-hydroxy-3-[(2'-methylphenyl)azo]-2,7-naphthalindi-sulfonsäure, 2-(2',4'-Dimethylphenylazo)-6-(4''-sulfophenylazo)-1,3-dihydroxybenzol, 1,4-Bis-(2',3'-dihydroxypropyl)amino-anthrachinon, 1-Methylamino-4-(2'-hydroxyethyl)amino-anthrachinon, 2-(2'-Aminoethyl)amino-anthrachinon, 2-Brom-4,8-diamino-6-(3'-trimethylammonium)-phenylamino-1,5-naphthochinon, 1-(2'-Sulfo-4'-methyl-phenyl)-amino-4-hydroxy-anthrachinon, 1,4-Diamino-anthrachinon, 1-Amino-2-sulfo-4-cyclohexylamino-anthrachinon, 1-Methylamino-4-aminopropylamino-anthrachinon, 1-Aminopropylamino-anthrachinon, 4',4'',4'''-Triamino-3-methyl-triphenylcarboniumchlorid, Bis-(4,4-Diethylaminophenyl)-4'-ethylamino-naphthyl-carboniumchlorid, Bis-(4,4-Dimethylaminophen)-4'-phenylamino-naphthyl-carbonsiumchlorid, 4,4-Bis-(N-Ethyl-3-sulfobenzyl)-amino-2''-sulfofuchsonium, 2,4-Dinitro-1-naphthol-7-sulfonsäure-Dinatriumsalz (Acid Yellow 1; Cl 10 316), 2-(2'-Chinolyl)-1H-indene-1,3(2H)-dion-monodisulfon-säure-Dinatriumsalz (Acid Yellow 3; Cl 47 005), 4,5-Dihydro-5-oxo-1-(4'-sulfophenyl)-4-[(4''-sulfo-phenyl) azo]-1H-pyrazol-3-carbonsäure-Trinatriumsalz (Acid Yellow 23; Cl 19 140), 3',6'-Dihydroxyspiro[isobenzofuran-1(3H), 9'(9H)-xanthen]-3-on (Acid Yellow 73; Cl 45 350:1), 5-[2',4'-Dinitrophenyl)amino]-2-(phenylamino)-benzol-sulfonsäure-Natriumsalz (Acid Orange 3; Cl 10 385), 4-[(2',4'-Dihydroxyphenyl)azo]-benzolsulfonsäure-Natrium-salz (Acid Orange 6; Cl 14 270), 4-[2'-Hydroxy-1'-naphthyl)azo]-benzolsulfonsäure-Natriumsalz (Acid Orange 7; Cl 15 510), 4-[[3'-[(2'',4''-Dimethylphenyl)azo]-2',4'-dihydroxyphenyl]azo]-benzolsulfonsäure-Natriumsalz (Acid Orange 24;Cl 20 170), 4-Hydroxy-3-[(4'-sulfo-1'-naphthyl)azo]-1-naphthalin-sulfonsäure-Dinatriumsalz (Acid Red 14; Cl 14 720), 7-Hydroxy-8-[(4'-sulfo-1'-naphthyl)azo]-1,3-naphthalin-disulfonsäure-Trinatriumsalz (Acid Red 18; Cl 16 255), 3-Hydroxy-4-[(4'-sulfo-1'-naphthyl)azo]-2,7-naphthalin-disulfonsäure-Trinatriumsalz (Acid Red 27; Cl 16 185), 5-Amino-4-hydroxy-3-phenylazo-2,7-naphtalindisulfon-säure-Dinatriumsalz (Acid Red 33; Cl 17 200), 5-(Acetylamino)-4-hydroxy-3-[(2'-methylphenyl)azo]-2,7-naphthalindisulfonsäure-Dinatriumsalz (Acid Red 35; Cl 18 065), 3',6'-Dihydroxy-2',4',5',7'-tetraiodospiro-[isobenzo-furan-1(3H), 9'(9H)-xanthen]-3-on-Dinatriumsalz (Acid Red 51; Cl 45 430), 3,6-Bis-(diethylamino)-9-(2',4'-disulfophenyl)-xanthyliumhydroxid-Natriumslz (Acid Red 52; Cl 45 100), 7-Hydroxy-8-[[4'-(phenylazo)phenyl]azo]-1,3-naphthalindisulfonsäure-Dinatriumsalz (Acid Red 73; Cl 27 290), 2',4',5',7'-Tetrabromo-3',6'-dihydroxyspiro[isobenzo-furan-1(3H),9'(9H)-xanthen]-3-on-Dinatriumsalz (Acid Red 87; Cl 45 380), 2',4',5',7'-Tetrabromo-4,5,6,7-tetrachloro-3',6'-dihydroxyspiro[isobenzofuran-1(3H),9'(9H)-xanthen]-3-on-Dinatriumsalz (Acid Red 92; Cl 45 410), 3',6'-Dihydroxy-4',5'-dijodospiro[isobenzofuran-1(3H),9'(9H)-xanthen]-3-on-Dinatriumsalz (Acid Red 95; Cl 45 425), Acid Red 195; Acid Blue 9 (Cl 42 090), 2,2'-[(9,10-Dihydro-9,10-dioxo-1,4-anthracendiyl)-diimino]-bis-(5-methylbenzolsulfonsäue)-Dinatriumsalz (Acid Green 25; Cl 61 570), N-[4-[[4'-(Dimethylamino)phenyl]-(2''-hydroxy-3'',6''-disulfo-1''-naphthyl)methylen]-2,5-cyclohexadien-1-yliden]-N-methylmethanaminiumhydroxid (Acid Green 50; Cl 44 090), N-[4-[[4'-Diethylamino)phenyl]-(2'',4''-disulfophenyl)-methylen]-2,5-cyclohexadien-1-yliden]-N-ethylethan- aminiumhydroxid-Natriumsalz (Acid Blue 1; Cl 42 045), N-[4-[[4'-Diethylamino)phenyl]-(5''-hydroxy-2'',4''-disulfo-phenyl)methylen]-2,5-caclohexadien-1-yliden]-N-ethyl-ethanaminiumhydroxid-Calciumsalz (Acid Blue 3; Cl 42 051), 1-Amino-4-(cyclohexylamino)-9,10-dihydro-9,10-dioxo-2-anthracensulfonsäure-Natrium-salz (Acid Blue 62; Cl 62 045), 2-(1',3'-Dihydro-3'-oxo-5'-sulfo-2'H-indol-2'-yliden)-2,3-dihydro-3-oxo-1H-indol-5-sulfonsäure-Dinatriumsalz (Acid Blue 74; Cl 73 015), 9-(2'-Carboxyphenyl)-3-[(2''-methylphenyl)amino]-6-[(2'''-methyl-4'''-sulfophenyl)amino)]-xanthylium-hydroxid-Natriumsalz (Acid Violet 9; Cl 45 190), 2-[(9',10'-Dihydro-4'-hydroxy-9',10'-dioxo-1'-anthracenyl)-amino]-5-methylbenzolsulfonsäure-Natriumsalz (Acid Violet 43; Cl 60 730), 3,3'-[Sulfonyl-bis(2-nitro-4,1-phenylen)imino]-bis-[6-phenylamino)-benzoldinatriumsulfonat] (Acid Brown 13; Cl 10 410), 4-Amino-5-hydroxy-3-[(4'-nitrophenyl)azo]-6-(phenylazo)-2,7-naphthalindisulfonsäure-Dinatriumsalz (Acid Black 1; Cl 20 470), 3-Hydroxy-4-[(2'-hydroxy-1'-naphthyl)azo]-7-nitro-1-naphthalinsulfonsäure-Natriumsalz (Acid Black 52; Cl 15 711), N-[4-[[4'-(Diethylamino)phenyl]-[4''-(ethylamino)-1''-naphthyl]-methylen]-2,5-cyclohexadien-1-yliden]-N-ethyl-ethanammoniumchlorid (Basic Blue 7; Cl 42 595), 4-[(4'-Aminophenyl)-(4'-imino-2',5'-cyclohexadien-1'-yliden)-methyl]-2-methyl-aminobenzol-Hydrochlorid (Basic Violet 14; Cl 42 510), 4-(Acetylamino)-5-hydroxy-6-[[7'-sulfo-4'-[(4''-Sulfo-phenyl)azo]-1'-naphthyl]azo]-1,7-naphthalin-disulfonsäure-Tetranatriumsalz (Brilliant Black 1; Cl 28 440), [8-(p-Aminophenyl)azo]-7-hydroxy-2-naphthyl]-tri-methylammoniumchlorid (Basic Brown 16; Cl 12 250), [8-[4'-Amino-2'-nitrophenyl)azo]-7-hydroxy-2-naphthyl]-trimethylammoniumchlorid (Basic Brown 17; Cl 12 251), 7-Hydroxy-8-[(2'-methoxyphenyl)azo]-N,N,N-trimethyl-2-naphthylammoniumchlorid (Basic Red 76; Cl 12 245), 3-[(4'-Amino-6'-bromo-5',8'-dihydro-1'-hydroxy-8'-imino-5'-oxo-2'-naphthyl)amino]-N,N,N-trimethylammonium-chlorid (Basic Blue 99; Cl 56 059) und 4-(3'-Trimethylammoniumphenylazo)-N-phenyl-3-methyl-pyrazolon(5) (Basic Yellow 57, Cl 12 719), eingesetzt werden.

Besonders bevorzugt werden kationische Farbstoffe, beispielsweise [8-(p-Aminophenyl)azo]-7-hydroxy-2-naphthyl]-tri-methylammoniumchlorid (Basic Brown 16, Cl 12 250), [8-[4'-Amino-2'-nitrophenyl)azo]-7-hydroxy-2-naphthyl]-trimethylammoniumchlorid (Basic Brown 17, Cl 12 251), 7-Hydroxy-8-[(2'-methoxyphenyl)azo]-N,N,N-trimethyl-2-naphthylammoniumchlorid (Basic Red 76, Cl 12 245), 3-[(4'-Amino-6'-bromo-5',8'-dihydro-1'-hydroxy-8'-imino-5'-oxo-2'-naphthyl)amino]-N,N,N-trimethylammonium-chlorid (Basic Blue 99, Cl 56 059), N-[4-[[4'-(Diethylamino)phenyl]-[4''-(ethylamino)-1''-naphthyl]-methylen]-2,5-cyclohexadien-1-yliden]-N-ethyl-ethanammoniumchlorid (Basic Blue 7, Cl 42 595), 4-(3'-Trimethylammoniumphenylazo)-N-phenyl-3-methyl-pyrazolon(5) (Basic Yellow 57, Cl 12 719) und 4-[(4'-Aminophenyl)-(4'-imino-2',5'-cyclohexadien-1'-yliden)-methyl]-2-methyl-aminobenzol-Hydrochlorid (Basic Violet 14, Cl 42 510), eingesetzt.

Als geeignete nichtionische Polymere sind insbesondere Homo- und Copolymerisate des Vinylpyrrolidons zu nennen, beispielsweise Polyvinylpyrrolidon, das Copolymerisat aus Vinylpyrrolidon und Vinylcaprolactam, das Copolymerisat aus Vinylpyrrolidon und Vinylacetat oder das Copolymerisat aus Vinylpyrrolidon und Vinylcaprolactam und Dimethylaminoethylmethacrylat, wobei Polyvinylpyrrolidon und das Vinylpyrrolidon/Vinylacetat-Copolymerisat besonders bevorzugt sind.

Als geeignete kationische Polymere können insbesondere mit Diethylsulfat quaternisierte Copolymerisate aus Vinylpyrrolidon und Dimethylaminoethylmethacrylat (Polyquaternium-11), mit Dimethylsulfat quaternisierte Homopolymerisate von Dimethylaminoethylmethacrylat (Polyquaternium-36), Poly(dimethyldiallylammoniumchlorid) (Polyquaternium-6), Kationische Cellulose(Polyquaternium-10) und Copolymerisate aus Vinylimidazoliumchlorid und Vinylpyrrolidon (Polyquaternium-16) genannt werden.

Besonders bevorzugt ist jedoch die Verwendung von nichtionischen Polymeren, insbesondere dem Polyvinylpyrrolidon und dem Vinylpyrrolidon/Vinylacetat-Copolymerisat.

Die Einsatzmenge der Polymere beträgt vorzugsweise 0,01 bis 10 Gewichtsprozent, wobei eine Menge von 0,2 bis 3 Gewichtsprozent besonders bevorzugt ist.

Als nichtionische Tenside können sowohl lineare als auch verzweigte Ethoxilate von $(C_{10} - C_{20})$-Fettalkoholen mit einem Ethoxilierungsgrad von 1 bis 100 verwendet werden. Weiterhin ist der Einsatz von Ethoxilaten natürlicher Öle und Fette, wie zum Beispiel Rizinusöl oder Sojaöl, sowie von Alkylpolyglucosiden möglich.

Als anionische Tenside können Alkalisalze von Fettsäuren, Fettalkoholsulfate und Fettalkoholethersulfate, insbesondere Laurylsulfate und Laurylethersulfate, Fettsäureisethionate und Fettsäuretauride eingesetzt werden.

Vorzugsweise werden als Tenside der Komponente (b) Laurylsulfate oder Laurylethersulfate, alleine oder in Kombination mit Rizinusölethoxilaten, eingesetzt.

Die Gesamtmenge an Tensiden beträgt in dem erfindungsgemäßen Mittel vorzugsweise 0,01 bis 10 Gewichtsprozent.

Weiterhin kann das erfindungsgemäße Haarfärbemittel alle für derartige Zubereitungen bekannten und üblichen Zusatzstoffe, beispielsweise Parfümöle; Komplexbildner; Wachse; Konservierungsstoffe, kosmetische Harze; Verdicker; Alginate; Guar Gum; haarpflegende Substanzen, wie zum Beispiel Panthenol oder Lanolinderivate; enthalten. Die erwähnten Bestandteile werden in den für solche Zwecke üblichen Mengen verwendet, zum Beispiel die Pflegestoffe in einer Menge von 0,1 bis 5 Gewichtsprozent.

Das erfindungsgemäße Haarfärbemittel kann neben Wasser weitere Lösungsmittel, wie zum Beispiel aliphatische Alkohole, insbesondere Ethanol oder Isopropanol, oder Glykolether, insbesondere 1,2-Propandiol, enthalten, wobei der Wassergehalt in der Regel etwa 20 bis 95 Gewichtsprozent beträgt, während der Gehalt an den übrigen Lösungsmitteln bei etwa 5 bis 80 Gewichtsprozent liegt.

Das erfindungsgemäße Haarfärbemittel kann in Form einer wäßrigen oder wäßrig-alkoholischen Lösung, einer Creme, eines Geles, einer Emulsion oder eines Aerosolschaumes vorliegen, wobei das Haarfärbemittel sowohl in Form eines Einkomponentenpräparates als auch in Form eines Mehrkomponentenpräparates konfektioniert sein kann.

Das erfindungsgemäße Mittel gelangt je nach gewünschter Farbintensität in unverdünnter Form oder nach einer 1:1- bis 1:10 - Verdünnung mit Wasser zur Anwendung.

Das Haarfärbemittel wird auf das gewaschene und handtuchtrockene Haar in einer für die Haarfärbung ausreichenden Menge aufgetragen und sodann einwirken gelassen. Je nach Applikationsart kann das Haar anschließend geföhnt werden, oder unter Wärmeeinwirkung (30 bis 50° Celsius) getrocknet werden, wobei das angewandte Produkt im Haar verbleibt.

Es ist jedoch auch möglich, das Haarfärbemittel direkt auf das trockene Haar zu geben und danach in der vorstehend beschriebenen Weise weiter zu verfahren.

Die nachfolgenden Beispiele sollen den Gegenstand der vorliegenden Erfindung näher erläutern ohne diesen hierauf zu beschränken.

**Beispiele**

**Beispiel 1:** Haartönungsmittel

| | |
|---|---|
| 3,00 g | Vinylpyrrolidon/Vinylacetat-Copolymer (Luviskol® VA 64 der Firma BASF/Ludwigshafen, BRD) |
| 1,00 g | Natriumlaurylsulfat |
| 0,50 g | Parfüm |
| 0,01 g | Basic Blue 7 |
| 0,05 g | Basic Brown 16 |
| 30,00 g | Ethanol |
| 65,44 g | Wasser |
| 100,00 | g |

Das Haartönungsmittel wird gleichmäßig im nassen Haar verteilt. Anschließend wird das Haar zur Frisur gelegt und getrocknet, wobei das Haartönungsmittel im Haar verbleibt.

Es wird eine helle, natürliche Braunfärbung der Haare erzielt. Nach einmaligem Waschen mit einem Shampoo wird eine deutlich hellere Braunfärbung erhalten. Nach zwei- bis dreimaliger Haarwäsche ist die Haarfärbung vollständig ausgewaschen.

**Beispiel 2:** Haartönungsmittel

| | | |
|---|---|---|
| 3,00 g | Polyvinylpyrrolidon (Luviskol® K90 der Firma BASF/Ludwigshafen, BRD) | |
| 0,50 g | Ethoxiliertes Rizinusöl, 40 Mol Ethylenoxid im Molekül (Cremophor® EL der Firma BASF/Ludwigshafen, BRD) | |
| 0,50 g | Parfüm | |
| 0,01 g | Basic Blue 7 | |
| 0,05 g | Basic Brown 16 | |
| 60,00 g | Isopropanol | |
| 35,94 g | Wasser | |
| 100,00 g | | |

Das Haartönungsmittel wird gleichmäßig in das nasse Haar eingearbeitet. Anschließend wird das Haar gefönt, wobei das Haartönungsmittel im Haar verbleibt.

Es wird eine helle, natürliche Braunfärbung der Haare erzielt. Nach einmaligem Waschen mit einem Shampoo ist diese Färbung deutlich aufgehellt. Nach zwei- bis dreimaliger Haarwäsche ist die Haarfärbung vollständig ausgewaschen.

Alle Prozentangaben stellen, soweit nicht anders angegeben, Gewichtsprozente dar.

**Patentansprüche**

1. Mittel zum Färben und Tönen von Haaren auf der Basis von einem oder mehreren direktziehenden Farbstoffen, dadurch gekennzeichnet, daß es (a) mindestens ein nichtionisches und/oder kationisches Polymer und (b) mindestens ein anionisches Tensid oder eine Mischung aus mindestens einem anionischen und mindestens einem nichtionischen Tensid enthält.

2. Mittel nach Anspruch 1, dadurch gekennzeichnet, daß das Tensid der Komponente (b) ausgewählt ist aus Laurylsulfaten, Laurylethersulfaten und Kombinationen von Laurylsulfaten und/oder Laurylethersulfaten mit Rizinusölethoxilaten.

3. Mittel nach Anspruch 1 oder 2, dadurch gekennzeichnet, daß das Tensid der Komponente (b) in einer Menge von 0,01 bis 10 Gewichtsprozent enthalten ist.

4. Mittel nach einem der Ansprüche 1 bis 3, dadurch gekennzeichnet, daß als direktziehender Farbstoff ein kationischer Farbstoff verwendet wird.

5. Mittel nach Anspruch 4, dadurch gekennzeichnet, daß der kationische Farbstoff ausgewählt ist aus [8-(p-Aminophenyl)azo]-7-hydroxy-2-naphthyl]-trimethylammoniumchlorid, [8-[4'-Amino-2'-nitrophenyl)azo]-7-hydroxy-2-naphthyl]-trimethyl-ammoniumchlorid, 7-Hydroxy-8-[(2'-methoxyphenyl)azo]-N,N,N-trimethyl-2-naphthylammoniumchlorid, 3-[(4'-Amino-6'-bromo-5',8'-dihydro-1'-hydroxy-8'-imino-5'-oxo-2'-naphthyl)amino]-N,N,N-trimethylammonium-chlorid, N-[4-[[4'-(Diethylamino)phenyl]-[4''-(ethylamino)-1''-naphthyl]-methylen]-2,5-cyclohexadien-1-yliden]-N-ethyl-ethanammoniumchlorid , 4-(3'-Trimethylammoniumphenylazo)-N-phenyl-3-methyl-pyrazolon(5) und 4-[(4'-Aminophenyl)-(4'-imino-2',5'-cyclohexadien-1'-yliden)-methyl]-2-methyl-aminobenzol-Hydrochlorid.

6. Mittel nach einem der Ansprüche 1 bis 5, dadurch gekennzeichnet, daß der direktziehende Farbstoff in einer Menge von 0,001 bis 5 Gewichtsprozent enthalten ist.

7. Mittel nach einem der Ansprüche 1 bis 6, dadurch gekennzeichnet, daß das Polymer der Komponente (a) ausgewählt ist aus Polyvinylpyrrolidon und dem Vinylpyrrolidon/Vinylacetat-Copolymerisat.

8. Mittel nach einem der Ansprüche 1 bis 7, dadurch gekennzeichnet, daß das Polymer der Komponente (a) in einer Menge von 0,01 bis 10 Gewichtsprozent enthalten ist.

9. Mittel nach einem der Ansprüche 1 bis 8, dadurch gekennzeichnet, daß es in Form einer einer wäßrigen oder wäßrig-alkoholischen Lösung, einer Creme, eines Geles, einer Emulsion oder eines Aerosolschaumes vorliegt.

10. Verfahren zum Färben und Tönen von Haaren, dadurch gekennzeichnet, daß man ein Mittel nach einem der Ansprüche 1 bis 9 auf das Haar in einer für die Haarfärbung ausreichenden Menge aufträgt, sodann einwirken läßt und abschließend trocknet.

11. Verfahren nach Anspruch 10, dadurch gekennzeichnet, daß das Haar vor der Anwendung des Mittels nach einem der Ansprüche 1 bis 10 gewaschen und mit einem Handtuch getrocknet wird.

12. Verfahren nach Anspruch 10 oder 11, dadurch gekennzeichnet, daß das Mittel nach einem der Ansprüche 1 bis 9 vor der Anwendung mit Wasser in einem Verhältnis von 1:1 bis 1:10 verdünnt wird.

Europäisches
Patentamt

**EUROPÄISCHER RECHERCHENBERICHT**

Nummer der Anmeldung

EP 97 11 7920

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | DE 32 46 747 A (HENKEL)<br>* Ansprüche 1,2 *<br>* Seite 6, Zeile 5-20 *<br>--- | 1-3,7-10 | A61K7/13 |
| X | WO 93 25182 A (WELLA)<br>* Ansprüche 1,5-9 *<br>* Seite 7, Absatz 2 *<br>--- | 1-3,6-10 | |
| X | EP 0 531 943 A (KAO CORPORATION)<br><br>* Ansprüche 1,6 *<br>* Seite 2, Zeile 56 - Seite 3, Zeile 1 *<br>* Seite 3, Zeile 33-56 *<br>* Seite 4, Zeile 7 *<br>* Beispiele 1,2 *<br>--- | 1-3,6,9, 10 | |
| X | DE 295 04 690 U (KAO CORPORATION)<br><br>* Ansprüche 1,6,7 *<br>* Seite 3, Absatz 2 *<br>* Seite 8, Absatz 6 *<br>* Seite 9, Absatz 4 *<br>* Beispiel 2 *<br>----- | 1-4,6,9, 10 | |

RECHERCHIERTE
SACHGEBIETE (Int.Cl.6)

A61K

Der vorliegende Recherchenbericht wurde für alle Patentansprüche erstellt

| Recherchenort | Abschlußdatum der Recherche | Prüfer |
|---|---|---|
| DEN HAAG | 20.April 1998 | Peeters, J |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
 
& : Mitglied der gleichen Patentfamilie, übereinstimmendes
    Dokument

EPO FORM 1503 03.82 (P04C03)